# DEMANDE DE BREVET EUROPEEN

(11) **EP 0 579 885 A1**
(43) Date de publication de la demande: **26.01.1994**
(21) Numéro de dépôt: 92420252.6
(22) Date de dépôt: 24.07.1992
(51) Int. Cl.: A61B 5/00, A61B 17/36

(54) **Procédé et dispositif d'évaluation de l'état de congélation d'un corps biologique**

(71) Demandeur: DIXWELL, F-69360 St. Symphorien d'Ozon (FR)
(72) Inventeur: Aguettant, Jean, F-69005 Lyon (FR); Petitjean, Philippe, F-69124 Colombier Saugnieu (FR)
(74) Mandataire: Bratel, Gérard

(57) **Abrégé**

Le procédé, et le dispositif pour sa mise en oeuvre, s'appliquent plus particulièrement au refroidissement jusqu'à congélation d'un corps biologique (1), notamment en cryochirurgie.

L'état de congélation du corps biologique (1) est évalué par la mesure de la réflexion et/ou de la transmission d'un faisceau lumineux injecté dans ce corps. Le faisceau, émis par une source de lumière (5), est propagé par une fibre optique (4) dont l'extrémité est implantée sur le corps (1) notamment au moyen d'une aiguille (6). Une libre optique réceptrice (7), aussi implantée sur ce corps, capte la fraction réfléchie ou transmise du faisceau, et la dirige vers des moyens récepteurs (8) qui traduisent cette fraction sous forme de signal électrique. A partir du traitement de ce signal, l'état de congélation est indiqué par un afficheur (14) et un avertisseur sonore (15).

## Description

La présente invention concerne un procédé d'évaluation de l'état de congélation d'un corps biologique, ainsi qu'un dispositif pour la mise en oeuvre de ce procédé.

Le procédé et le dispositif, objets de la présente invention, s'appliquent en particulier au refroidissement jusqu'à congélation d'un corps biologique notamment dans le domaine médical et plus particulièrement en cryochirurgie.

La cryochirurgie est un procédé de destruction tissulaire "in situ", par congélation localisée. Cette congélation peut être réalisée par divers procédés, qui se différencient notamment par la source frigorigène utilisée : azote liquide, protoxyde d'azote, anhydride carbonique, ...

Il est essentiel pour le clinicien de contrôler l'étendue de la cryolésion induite et de délimiter au mieux le volume "cible", afin de réduire à une marge acceptable l'extension de la cryolésion aux tissus voisins constitués de cellules saines.

Les procédés de contrôle utilisés dans la pratique cryochirurgicale font souvent appel à un principe thermique, avec mesure de température, par implantation d'un ou plusieurs thermocouples ; il s'agit d'un principe simple, mais celui-ci s'avère insuffisamment fiable, imprécis et trop ponctuel.

Un autre principe, de plus en plus utilisé, est la mesure de l'impédance bioélectrique. Cette mesure est réalisée au moyen d'électrodes implantées dans le corps et reliées à un impédancemètre. Des procédés et dispositifs rattachés à ce principe ont été déjà décrits dans le brevet français N° 2 289 157 et dans son certificat d'addition N° 2 333 483, et plus récemment dans la demande de brevet d'invention N° 91 00464 déposée le 11 Janvier 1991 au nom de la Demanderesse. Ces procédés et dispositifs sont basés sur le fait que, dans un milieu biologique, la résistance ou l'impédance électrique du milieu augmente avec le niveau de congélation. La congélation s'effectue jusqu'à l'obtention de l'impédance correspondant à la cristallisation eutectique de l'eau extracellulaire.

Ce principe de mesure électrique, plus fiable que le principe thermique, a indéniablement fait progresser et banaliser l'acte cryochirurgical. Toutefois, il conserve encore quelques inconvénients : risque permanent de perturbation de la mesure par la présence d'une capacité parasite (d'où nécessité d'emploi de câbles blindés), sensibilité aux champs électriques voisins, risque de polarisation, etc...

La présente invention vise à fournir un procédé perfectionné et original d'évaluation de l'état de congélation d'un corps biologique permettant un contrôle précis de lacryolésion en éliminant l'ensemble des inconvénients présentés par les procédés précédemment évoqués, et notamment en évitant toute perturbation par des phénomènes électriques.

A cet effet, le procédé objet de l'invention consiste, essentiellement, à évaluer l'état de congélation d'un corps biologique par la mesure de la réflexion et/ou de la transmission d'un faisceau lumineux injecté dans le corps biologique. Ainsi, l'invention propose un principe optique, et non plus thermique ou électrique, pour l'évaluation de l'état de congélation, le procédé préconisé pouvant se justifier comme suit :

Un tissu biologique est essentiellement composé d'eau contenant des solutés. Lorsque l'on abaisse la température du tissu biologique, on observe un changement de phase de ce tissu. Il se produit initialement une formation de glace extracellulaire, la partie liquide se concentrant en solutés. La cristallisation du milieu extracellulaire se poursuit, avec l'abaissement de température, jusqu'à cristallisation complète du milieu extracellulaire, ce phénomène étant appelé "congélation eutectique" : l'état du milieu est alors stable si la température est maintenue ou continue d'être abaissée. Le processus s'achève par la cristallisation intracellulaire.

Lorsque l'état de cristallisation extracellulaire complète est atteint, la dose léthale de froid a été administrée : la nécrose du volume cible tissulaire sera totale. L'injection d'un faisceau lumineux permet, en combinaison avec des moyens de mesure appropriés d'évaluer l'état cristallin du tissu biologique en cours de congélation, et d'arrêter la congélation pour un état correspondant a la cristallisation complète du milieu extracellulaire. Le milieu se trouvant en quelque sorte "éclairé" par le faisceau lumineux, l'importance de la réflexion ou de la transmission de ce faisceau par le milieu évolue de façon caractéristique au fur et à mesure du refroidissement, en étant d'abord croissante puis en se stabilisant au moment où l'état de cristallisation extracellulaire est atteint. La détermination de l'importance relative de la réflexion ou de la transmission, exprimée par exemple en décibels par rapport au niveau de la réflexion ou de la transmission avant le début de la congélation, et l'analyse des variations de cette réflexion ou transmission, donnent ainsi une indication significative de l'état de congélation du corps biologique. Comme on le comprend aisément, le principe de l'invention garantit une isolation électrique totale.

Selon un premier mode de mise en oeuvre de cette invention, on détermine et on exploite un coefficient de réflexion du faisceau injecté dans le corps biologique, comme indication significative de l'état de congélation de ce corps, notamment par rapport à une valeur initiale de la réflexion, mesurée avant le refroidissement dudit corps.

Selon un second mode de mise en oeuvre de la même invention, on détermine et on exploite un coefficient de transmission du faisceau injecté dans le corps biologique, comme indication significative de l'état de congélation de ce corps, notamment par rapport à une valeur initiale de la transmission, mesurée avant le refroidissement dudit corps.

Le dispositif selon l'invention, pour la mise en oeuvre du procédé d'évaluation de l'état de congélation d'un corps biologique précédemment défini, comprend essentiellement en combinaison : une source de lumière apte à émettre un faisceau lumineux, une fibre optique émettrice apte à propager le faisceau lumineux émis vers le corps biologique, une extrémité de cette fibre étant apte à être implantée sur ledit corps, une fibre optique réceptrice également apte à être implantée par une extrémité sur ce corps biologique et apte à capter la fraction réfléchie ou transmise dudit faisceau, et des moyens récepteurs de la fraction réfléchie ou transmise du faisceau, aptes à traduire cette fraction sous forme de signal électrique. Le dispositif comprend encore, avantageusement, des moyens de traitement de ce signal, aptes à évaluer la fraction de faisceau lumineux réfléchie ou transmise pour le corps biologique, des moyens d'affichage d'une valeur représentative de l'état de congélation et des moyens avertisseurs, par exemple sonores, de l'atteinte d'un certain état de congélation.

Selon une première forme de réalisation de ce dispositif, plus particulièrement adaptée à la mesure de la réflexion du faisceau lumineux par le corps biologique, la fibre optique émettrice et la fibre optique réceptrice ont leurs extrémités respectives réunies dans une même aiguille, apte à être implantée dans le corps biologique. Les fibres émettrice et réceptrice peuvent ainsi être accolées sur toute leur longueur, et la réalisation pratique correspondante est particulièrement simple.

Dans une variante de ce dispositif, également adaptée à la réflexion du faisceau lumineux par le corps biologique, la fibre optique émettrice et la fibre optique réceptrice ont leurs extrémités respectives raccordées, par un connecteur ou un coupleur, à une fibre optique unique commune à l'émission et à la réception insérée dans une aiguille apte à être implantée dans le corps biologique.

Selon une autre forme de réalisation du dispositif objet de l'invention, plus spécialement destinée à la mesure de la transmission du faisceau lumineux par le corps biologique, la fibre optique émettrice et la fibre optique réceptrice ont leurs extrémités respectives séparées l'une de l'autre par une distance prédéterminée, ces extrémités aboutissant à des aiguilles respectives aptes à être implantées l'une et l'autre dans le corps biologique. Ainsi, les deux fibres restent distantes l'une de l'autre à leurs extrémités implantables, le faisceau lumineux étant injecté dans le corps par une aiguille et sa fraction transmise étant captée par une autre aiguille ; la distance entre ces aiguilles est choisie telle que le signal résiduel capté par la seconde fibre optique reste détectable. La mesure réalisable par ce dernier dispositif est plus "volumique" et moins "ponctuelle" que celle effectuée avec la première forme de réalisation.

De toute façon, l'invention sera mieux comprise à l'aide de la description qui suit, en référence au dessin schématique annexé représentant; à titre d'exemples non limitatifs, quelques formes d'exécution de ce dispositif d'évaluation de l'état de congélation d'un corps biologique :
Figure 1 est une vue d'ensemble, très schématique, d'un premier dispositif conforme à la présente invention, en cours d'utilisation ;
Figure 2 est un schéma synoptique des circuits électroniques du dispositif de figure 1 ;
Figure 3 est une vue similaire à figure 1, mais correspondant à une autre forme de réalisation de l'invention ;
Figures 4 et 5 sont des vues partielles du dispositif, illustrant des variantes de l'invention.

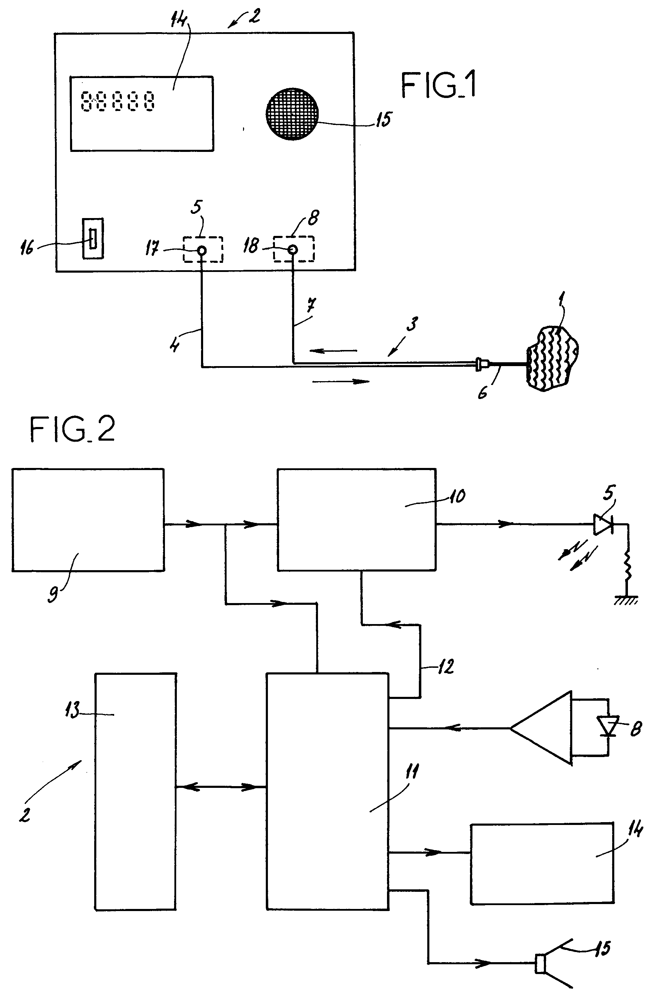

Sur la figure 1, le repère 1 désigne un tissu biologique, tel qu'une tumeur cutanée, soumis à une congélation réalisée elle-même par des moyens connus, non directement concernés par la présente invention et par conséquent non représentés. Le dispositif objet de l'invention se présente comme un appareil 2 avec boîtier, comportant une liaison 3 par fibres optiques avec le tissu biologique 1, l'appareil 2 étant raccordé à ce tissu 1 avant le début de la congélation, et le restant durant toute la congélation et ce jusqu'à l'arrêt de la congélation.

Cet appareil, pouvant être désigné comme un "réflectomètre cutané", est conçu pour injecter dans le tissu biologique 1 un faisceau lumineux, par une fibre optique 4 constituant un premier élément de la liaison 3. La fibre optique 4, partant d'un élément émetteur 5 de l'appareil 2, aboutit à une aiguille 6 prévue pour être implantée dans le tissu biologique 1. La réflexion du faisceau lumineux est captée par une autre libre optique 7, ressortant de la même aiguille 6 et constituant un second élément de la liaison 3. La libre optique 7 aboutit à un élément récepteur 8 de l'appareil 2 et transmet ainsi le faisceau réfléchi. Les fibres émettrice 4 et réceptrice 7 sont accolées, la liaison optique 3 se présentant comme un câble souple unique ; ces libres sont réalisables en matière synthétique ou en silice.

En se référant à la figure 2, montrant sous forme de schéma synoptique les circuits électroniques internes de l'appareil 2, celui-ci comprend un bloc d'alimentation électrique stabilisée 9, un régulateur de tension à commande numérique 10, un microcontrôleur 11 relié par une ligne de commande 12 au régulateur de tension 10, et une diode émettrice infrarouge de longueur d'onde par exemple égale à 850 nm alimentée par le régulateur de tension 10, cette diode constituant l'élément émetteur 5. Une photodiode, constituant l'élément récepteur 8, est reliée au microcontrôleur 11, auquel sont aussi raccordés une mémoire morte 13 contenant un programme d'exécution, un afficheur à cristaux liquides 14 et un ronfleur 15, ces derniers organes apparaissant également sur la figure 1 qui montre, en outre, l'interrupteur général 16 de mise en marche et d'arrêt de l'appareil 2.

Lors de l'utilisation du dispositif, mais avant refroidissement du tissu biologique 1, le faisceau lumineux émis par la diode 5 et se propageant le long de la libre optique 4 est injecté par l'aiguille 6 et il se disperse dans le tissu 1. La réflexion de ce faisceau est faible, et cette réflexion captée par l'autre libre optique 7 et détectée par la photodiode 8 est enregistrée comme valeur de référence initiale pour un calcul différentiel ultérieur.

La progression de la congélation du tissu 1 induit une augmentation de la réflexion, dont le rapport comparativement à la valeur de référence initialement enregistrée est un coefficient exprime par exemple en décibels. Lorsque cette réflexion n'augmente plus et devient stable, l'état de cristallisation extracellulaire est atteint. L'opérateur est averti de cet état par le ronfleur 15.

Grâce à son microcontrôleur 11, l'appareil 2 permet :
- d'asservir la puissance du faisceau lumineux émis par rapport à une valeur constante ;
- d'annoncer que la valeur de référence de la réflexion est prise et que l'opération peut commencer ;
- de détecter le seuil de cryodestruction (une fois que le coefficient de réflexion a dépassé le critère de cryodestruction) ;
- d'afficher en 14, au cours de l'opération de congélation, l'évolution du coefficient de réflexion ;
- d'activer le ronfleur 15 pour signaler que le seuil de cryodestruction est atteint.

L'ensemble constitué par les deux libres optiques 4 et 7 accolées en un câble souple, et par l'aiguille 6, réunissant les extrémités de ces deux fibres, forme une "sonde de mesure" que des connecteurs appropriés 17 et 18 permettent de raccorder au boîtier de l'appareil 2 au moment de l'utilisation du dispositif. L'aiguille 6 réunissant ici les extrémités des deux libres optiques 4 et 7 est une aiguille de type médical, dont le diamètre peut être compris par exemple entre 0,4 et 0,7 mm.

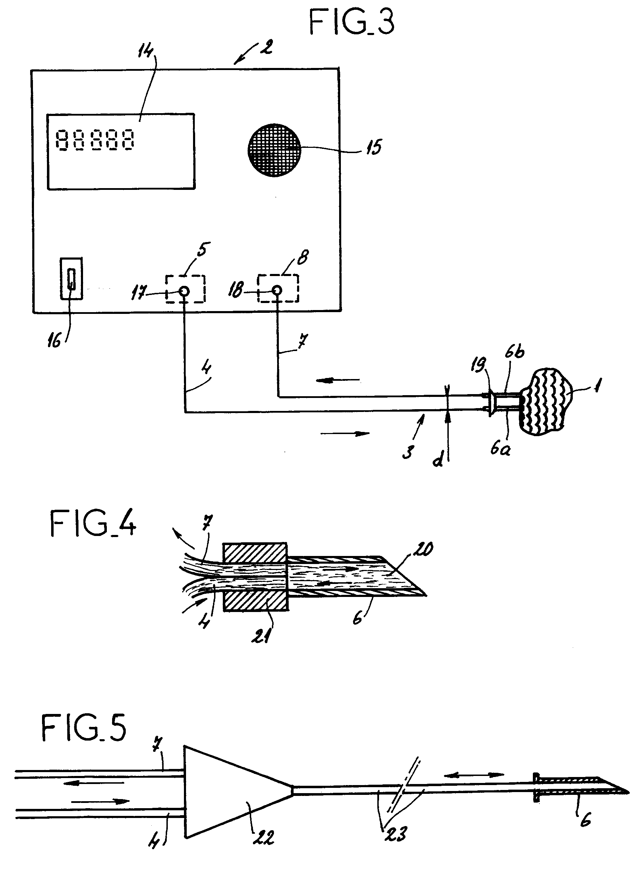

La figure 3 montre une autre forme de réalisation du dispositif, les éléments correspondant à ceux précédemment décrits y étant désignés par les mêmes repères et n'étant pas décrits une nouvelle fois. La différence réside dans le fait que les deux libres optiques 4 et 7, respectivement émettrice et réceptrice, sont ici isolées l'une de l'autre et aboutissent à deux aiguilles respectives 6a et 6b, distinctes l'une de l'autre. Les deux aiguilles 6a et 6b sont prévues pour être implantées dans le tissu biologique 1 à une distance d l'une de l'autre.

L'extrémité de la première libre optique 4 permet d'injecter le faisceau lumineux dans le tissu biologique 1. Ce faisceau se transmet au travers du tissu 1 et est capté par l'extrémité de la seconde libre optique 7. L'importance relative du faisceau capté est mesurée par l'appareil 2 et permet de définir un coefficient de transmission, analogue au coefficient de réflexion considéré dans la première forme de réalisation. L'appareil 2 analyse l'évolution du coefficient de transmission, traduisant l'état de congélation du tissu biologique 1, par des circuits électroniques réalisables aussi selon la figure 2.

En pratique, les extrémités respectives des deux libres optiques 4 et 7 sont réunies au moyen d'une embase commune 19, qui supporte les deux aiguilles 6a et 6b en fixant leur distance d. Ces deux aiguilles 6a et 6b, de type médical, contenant chacune une seule extrémité de libre optique, peuvent avoir chacune un diamètre compris par exemple entre 0,3 mm et 0,5 mm.

Dans l'exposé qui précède, on a distingué les libres optiques émettrice 4 et réceptrice 7. Dans le dispositif de la figure 1, la séparation des deux libres 4 et 7 peut s'avérer délicate, surtout pour la conception de l'aiguille 6. Pour éviter cette difficulté, on peut utiliser une seule libre optique au niveau de l'aiguille 6, des réalisations correspondantes étant illustrées aux figures 4 et 5.

Ainsi, dans la variante selon la figure 4, l'aiguille 6 renferme une libre optique unique 20, de faible longueur. Un connecteur 21, placé à l'extrémité arrière de l'aiguille 6, permet de raccorder la libre unique 20 de cette aiguille 6 à la fibre émettrice 4 et à la fibre réceptrice 7. On comprend que cette fibre unique 20 assure d'une part l'injection dans le corps biologique (non représenté) du faisceau lumineux reçu de la fibre émettrice 4, et d'autre part la captation de la fraction réfléchie du faisceau et sa transmission vers la libre réceptrice 7.

Selon une autre variante, montrée en figure 5, un coupleur pour fibres optiques 22 assure le raccordement entre la libre émettrice 4 et la libre réceptrice 7, d'une part, et une fibre optique 23 d'une certaine longueur, commune à l'émission et à la réception, qui se termine dans l'aiguille 6. Le coupleur 22 a la faculté de dissocier l'émission de la réception.

Dans le même ordre d'idées, on peut utiliser un signal optique pulsé, qui permet de différencier l'instant d'émission de l'instant de réception. Ainsi, l'émission lumineuse se lait sur une libre optique à un instant donné, et la réception peut se faire par la même libre à un instant légèrement différé. Ceci permet donc de confondre la fibre optique émettrice et la fibre optique réceptrice, sur toute leur longueur, en une libre optique unique, sous réserve que la source de lumière soit une source de lumière pulsée.

Comme il va de soi, l'invention ne se limite pas aux seules formes d'exécution de ce dispositif pour l'évaluation de l'état de congélation d'un corps biologique qui ont été décrites ci-dessus, à titre d'exemples ; elle en embrasse, au contraire, toutes les variantes de réalisation et d'application respectant le même principe. Ainsi, l'on ne s'éloignerait pas du cadre de l'invention :
- en utilisant un autre type de source de lumière, telle qu'un laser, pour l'émission du faisceau lumineux injecté dans le corps biologique, la longueur d'onde de l'émission lumineuse pouvant naturellement varier elle aussi ;
- en modifiant la réalisation des circuits électroniques de l'appareil et la forme sous laquelle les résultats de la mesure sont affichés ;
- en utilisant simultanément deux ou plusieurs couples de libres émettrice-réceptrice implantés sur le même corps biologique, pour avoir une meilleure "vue d'ensemble" de son état de congélation, chaque couple de libres ayant sa source lumineuse propre, ses moyens d'affichage et ses moyens avertisseurs de l'atteinte d'un certain état de congélation.

## Revendications

1. Procédé d'évaluation de l'état de congélation d'un corps biologique, applicable plus particulièrement au refroidissement jusqu'à congélation d'un corps biologique notamment en cryochirurgie, caractérisé en ce qu'il consiste essentiellement à évaluer l'état de congélation du corps biologique (1) par la mesure de la réflexion et/ou de la transmission d'un faisceau lumineux injecté dans le corps biologique.

2. Procédé d'évaluation de l'état de congélation d'un corps biologique, selon la revendication 1, caractérisé en ce que l'on détermine et exploite un coefficient de réflexion du faisceau lumineux injecté dans le corps biologique (1), en captant la fraction réfléchie de ce faisceau, comme indication significative de l'état de congélation de ce corps, notamment par rapport à une valeur initiale de la réflexion, mesurée avant le refroidissement dudit corps.

3. Procédé d'évaluation de l'état de congélation d'un corps biologique, selon la revendication 1, caractérisé en ce que l'on détermine et exploite un coefficient de transmission du faisceau lumineux injecté dans le corps biologique (1), en captant la fraction transmise de ce faisceau, comme indication significative de l'état de congélation de ce corps, notamment par rapport à une valeur initiale de la transmission, mesurée avant le refroidissement dudit corps.

4. Dispositif pour la mise en oeuvre du procédé d'évaluation de l'état de congélation d'un corps biologique selon l'une quelconque des revendications 1 à 3, caractérisé en ce qu'il comprend essentiellement, en combinaison : une source de lumière (5) apte à émettre un faisceau lumineux, une libre optique émettrice (4) apte à propager le faisceau lumineux émis vers le corps biologique (1), une extrémité de cette libre (4) étant apte à être implantée sur ledit corps (1), une fibre optique réceptrice (7) également apte à être implantée par une extrémité sur ce corps biologique (1) et apte à capter la fraction réfléchie ou transmise dudit faisceau, et des moyens récepteurs (8) de la fraction réfléchie ou transmise du faisceau, aptes à traduire cette fraction sous forme de signal électrique.

5. Dispositif d'évaluation de l'état de congélation d'un corps biologique, selon la revendication 4, caractérisé en ce que la source de lumière est constituée par une diode émettrice infrarouge (5).

6. Dispositif d'évaluation de l'état de congélation d'un corps biologique, selon la revendication 4 ou 5, caractérisé en ce que les moyens récepteurs sont constitués par une photodiode (8).

7. Dispositif d'évaluation de l'état de congélation d'un corps biologique, selon l'une quelconque des revendications 4 à 6, caractérisé en ce qu'il comprend encore des moyens de traitement (11,13) du signal fourni par les moyens récepteurs (8), ces moyens de traitement étant aptes à évaluer la fraction de faisceau lumineux réfléchie ou transmise par le corps biologique (1), ainsi que des moyens d'affichage (14) d'une valeur représentative de l'état de congélation et des moyens avertisseurs, par exemple sonores (15), de l'atteinte d'un certain état de congélation.

8. Dispositif d'évaluation de l'état de congélation d'un corps biologique, selon la revendication 7, caractérisé en ce que la source de lumière, telle que diode émettrice infrarouge (5), est alimentée par un régulateur de tension (10), et en ce que les moyens de traitement comprennent un microcontrôleur (11) relié par une ligne de commande (12) au régulateur de tension (10).

9. Dispositif d'évaluation de l'état de congélation d'un corps biologique, selon l'une quelconque des revendications 4 à 8, plus particulièrement adapté à la mesure de la réflexion du faisceau lumineux par le corps biologique (1), caractérisé en ce que la libre optique émettrice (4) et la libre optique réceptrice (7) ont leurs extrémités respectives réunies dans une même aiguille (6), apte à être implantée dans le corps biologique (1).

10. Dispositif d'évaluation de l'état de congélation d'un corps biologique, selon l'une quelconque des revendications 4 à 8, plus particulièrement adapté à la mesure de la réflexion du faisceau lumineux par le corps biologique (1), caractérisé en ce que la libre optique émettrice (4) et la libre optique réceptrice (7) ont leurs extrémités respectives raccordées par un connecteur ou un coupleur (21,22) à une libre optique unique (20, 23) commune à l'émission et à la réception, insérée dans une aiguille (6) apte à être implantée dans le corps biologique (1).

11. Dispositif d'évaluation de l'état de congélation d'un corps biologique, selon l'une quelconque des revendications 4 à 8, plus particulièrement adapté à la mesure de la réflexion du faisceau lumineux par le corps biologique (1), caractérisé en ce que la libre optique émettrice et la libre optique réceptrice sont con fondues, sur toute leur longueur, en une libre optique unique, la source de lumière (5) étant une source de lumière pulsée permettant de distinguer l'instant d'émission de l'instant de réception.

12. Dispositif d'évaluation de l'état de congélation d'un corps biologique, selon l'une quelconque des revendications 4 à 8, plus particulièrement adapté à la mesure de la transmission du faisceau lumineux par le corps biologique (1), caractérisé en ce que la libre optique émettrice (4) et la libre optique réceptrice (7) ont leurs extrémités respectives séparées l'une de l'autre par une distance prédéterminée (d), ces extrémités aboutissant à des aiguilles respectives (6a,6b) aptes à être implantées l'une et l'autre dans le corps biologique (1).

13. Dispositif d'évaluation de l'état de congélation d'un corps biologique, selon la revendication 12, caractérisé en ce que les extrémités respectives des deux libres optiques (4,7) sont réunies au moyen d'une embase commune (19), qui supporte les deux aiguilles (6a,6b) en fixant leur distance (d).
